# EUROPEAN PATENT SPECIFICATION

(11) **EP 2 987 799 B1**
(45) Date of publication and mention of the grant of the patent: **08.04.2020**
(21) Application number: 13882193.9
(22) Date of filing: 18.04.2013
(51) Int. Cl.: C07J 31/00, A61K 31/565, A61K 9/00, A61P 5/32, A61P 35/00

(54) **ESTER DERIVATIVE OF 7-ALPHA-[9-(4,4,5,5,5-PENTAFLUOROPENTYLSULPHINYL)NONYL]OESTRA-1,3,5(10)-TRIENE-3,17BETA-DIOL HAVING ANTITUMOUR ACTIVITY AND PREPARATION METHOD THEREOF**
ESTERDERIVATE VON 7-ALPHA-[9-(4,4,5,5,5-PENTAFLUORPENTYLSULPHINYL)NONYL]OESTRA-1,3,5(10)-TRIEN-3,17BETA-DIOL MIT ANTITUMORAKTIVITÄT UND HERSTELLUNGSVERFAHREN DAFÜR
DÉRIVÉ ESTER DU 7-ALPHA-[9-(4,4,5,5,5-PENTAFLUOROPENTYLSULPHINYL)NONYL] STRA-1,3,5(10)-TRIÈNE-3,17-BÊTA-DIOL POSSÉDANT UNE ACTIVITÉ ANTITUMORALE ET PROCÉDÉ DE PRÉPARATION CORRESPONDANT

(43) Date of publication of application: 24.02.2016
(73) Proprietor: Xi'an Libang Pharmaceutical Technology Co., Ltd., Xi'an, Shaanxi 710065 (CN)
(72) Inventor: JIAO, Yaqi, Shaanxi 710077 Xi'an (CN); WANG, Jiucheng, Shaanxi 710077 Xi'an (CN); HU, Renle, Shaanxi 710077 Xi'an (CN)
(74) Representative: Harrison IP Limited
(86) International application number: PCT/CN2013/074363
(87) International publication number: WO 2014/169462

(56) References cited:
- EP-A2- 0 138 504
- WO-A1-98/07740
- WO-A2-2011/066542
- CN-A- 1 690 073
- CN-A- 102 600 073
- DE-A1- 4 218 743
- US-A- 4 212 864
- US-A- 4 659 516
- US-A1- 2006 030 552
- DATABASE CA [Online] CHEMICAL ABSTRACTS SERVICE, COLUMBUS, OHIO, US; ZHONG, HUIJUAN ET AL: "Preparation of steroid derivatives as antimammary cancer", XP002760978, retrieved from STN Database accession no. 2005:1193077 -& WO 2005/105823 A1 (JIANGSU HANSOH PHARMACEUTICAL [CN]; ZHONG HUIJUAN [CN]; LUE AIFENG [CN) 10 November 2005 (2005-11-10)
- ADAMS J B ET AL: "Metabolism of lipoidal derivatives of estradiol-17beta in human mammary cancer tissue and cell lines", JOURNAL OF STEROID BIOCHEMISTRY AND MOLECULAR BIOLOGY, ELSEVIER SCIENCE LTD., OXFORD, GB, vol. 39, no. 5, 1 November 1991 (1991-11-01), pages 751-758, XP023550165, ISSN: 0960-0760, DOI: 10.1016/0960-0760(91)90376-G [retrieved on 1991-11-01]
- DATABASE CA [Online] CHEMICAL ABSTRACTS SERVICE, COLUMBUS, OHIO, US; JANOCKO, LAURA ET AL: "The interaction of C-17 esters of estradiol with the estrogen receptor", XP002760982, retrieved from STN Database accession no. 1984:186010 & JANOCKO, LAURA ET AL: "The interaction of C-17 esters of estradiol with the estrogen receptor", ENDOCRINOLOGY , 114(4), 1180-6 CODEN: ENDOAO; ISSN: 0013-7227, 1984, DOI: 10.1210/ENDO-114-4-1180 10.1210/ENDO-114-4-1180
- FERGUSON, J.R. ET AL.: 'Putative metabolites of fulvestrant, an estrogen receptor downregulator. Improved glucuronidation using trichloroacetimidates.' JOURNAL OF THE CHEMICAL SOCIETY, PERKIN TRANSACTIONS 1. vol. 22, 2001, pages 3037 - 3041, XP055279714
- WANG TING: 'Study on the Synthetic Technique of Pure Antiestrogen Agent Fulvestrant' SCIENCE -ENGINEERING (A), CHINA MASTER'S THESES FULL-TEXT DATABASE 15 January 2009, page B016-124, XP008181252

## Description

### Technical Field

The invention belongs to the field of medicine, specifically relates to a preparation method of compounds of general Formula A, and more specifically relates to the ester derivatives of 7-α-[9-(4,4,5,5,5-pentafluoropentylsulfinyl)nonyl]-estra-1,3,5(10)-triene-3,17β -diol and preparation method thereof.

### Background Art

7-α-[9-(4,4,5,5,5-pentafluoropentylsulfinyl)nonyl]-estra-1,3,5(10)-triene-3,17β-diol, also referred to as fulvestrant, having a general Formula B, is a novel estrogen receptor blocking agent in the treatment of postmenopausal advanced breast cancer which fails to respond to anti-estrogen therapy and which is estrogen receptor positive.

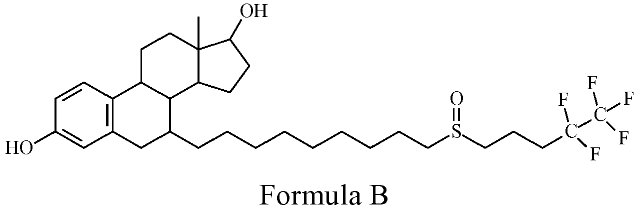

The most important feature of breast cancer is that its occurrence and development associates with the estrogen level and metabolism thereof in vivo. Studies have shown that estrogen receptor (ER) was found in tumor cells of many patients with breast cancer, and tumor growth was stimulated by estrogen. Thus, one of the main methods for treating breast cancer is reducing the concentration of estrogen or blocking the binding of estrogen to its receptor to inhibit the growth and reproduction of tumor cells. Fulvestrant can competitively binding to estrogen receptors, with affinity similar to estradiol; it can also block the receptors, inhibit the binding of estrogen, stimulate deformation of receptors, and reduce ER concentration to destroy tumor cells. Fulvestrant can down-regulate ER protein in human breast cancer cells, down-regulate ER in tumor cells, and minimize tumor growth. Since fulvestrant does not change the condition of existing tumor ER and does not affect the generation of new ER, the tumor continues to be "programmed" as ER positive. In this way, fulvestrant continues to have therapeutical effects. Its greatest advantage is that it does not have partial agonistic action and estrogen-like activity of common antiestrogen drugs.

Currently, many commercial available preparations of fulvestrant use oil as an excipient for the following two reasons. On the one hand, fulvestrant, which is of poor stability and easy to degrade, is generally stored at -20°C, and should not be stored at room temperature for too long, otherwise its purity would be affected. Although the mechanism of its degradation is not clear, it is generally believed that the main reason for affecting its stability lies in the presence of -OH at positions C-3 and C-17. Meanwhile, the presence of -OH at 3- and 17- positions increases the polarity of drugs and the stimulation of drugs on the gastrointestinal tract, thus it can only be prepared into injection.

On the other hand, like other steroids, fulvestrant, which is difficult to be formulated due to certain physical properties, is a molecule with high lipophilicity and extremely low water solubility of about 10ng/mL. Its solubility is provided in US5183514 and CN1394141A (mg/mL, 25°C)(water 0.001, peanut oil 0.45, sesame oil 0.58, castor oil 20, Migloyl 810 3.06, Migloyl 812 2.72, ethyl oleate 1.25, benzyl benzoate 6.15, isopropyl myristate 0.80, Span 85 3.79, ethanol>200, benzyl alcohol>200). The 17-n-propionate ester of fulvestrant, and its use in treating mammary cancer, is described in Zhong, Huijuan et al., "Preparation of steroid derivatives as antimammary cancer" WO-A-2005/105823, see also the abstract thereof in Chemical abstracts service (Columbus, Ohio, US), database accession no. 2005:1193077.

It can be seen that, even in castor oil with the maximum solubility, it is impossible to provide a concentration of fulvestrant that meets clinical requirement for administration. Therefore, many fulvestrant preparations in the marketplace not only use oil as solvent, but also add other excipients, such as ethanol, benzyl benzoate, benzyl alcohol and the like, to facilitate solubilizing. In this way, it can be formulated into intramuscularly injectable injections with content not less than 45mg/mL, which can maintain effective plasma concentration (2.5ng/mL) for 2 weeks. However, the addition of such solvents may increase the risk of precipitation of the drug in the preparations and cause irritation at injection sites.

Thus it can be seen that, the problem to be solved in prior art is how to make structural improvements to fulvestrant, especially to -OH at positions C-3 and C-17, so as to reduce irritation to human body and increase its lipophilicity, thereby making it more easily to be formulated into preparations for human use, while maintain its inhibition effect on cancer cell.

### Summary of Invention

Therefore, one object of the present invention is to make improvement to -OH at C-3 and C-17 positions of fulvestrant structure, and esterify fulvestrant into ester (including carboxyl carbon) compounds having 2 to 22 carbon atoms at C-17 position and ester (including carboxyl carbon) compounds having 2 to 4 carbon atoms at C-3 position, so as to increase the drug stability and its solubility in lipophilic solvents.

The object of the present invention is achieved by the following technical solutions:

The present invention provides a compound of Formula A: 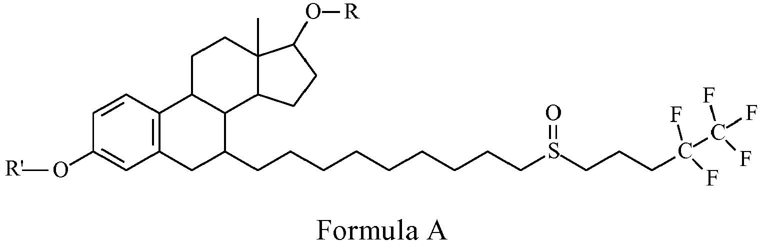 wherein,
substituent R' is selected from H, butyryl or alkenoyl having 2 to 4 carbon atoms,
substituent R is selected from alkanoyl or alkenoyl having 11 to 22 carbon atoms.

Preferably, said substituent R is selected from undecanoyl, hexadecanoyl, docosanoyl or 2-[(3',3')-dimethyl-1'-methyl]butyl-5-methyl-(7,7)-dimethyl-octanoyl.

Preferably, said substituent R is alkenoyl containing 1 to 6 carbon-carbon double bonds, wherein said carbon-carbon double bonds can either be distributed in the main chain, or in the branched chain.

Preferably, said substituent R is selected from undec-2-enoyl, eicosa-5,8,11,14,17-pentaenoyl and docosa-(4,7,10,13,16,19)-hexaenoyl.

Preferably, said substituent R is selected from 2-[(3',3')-dimethyl-1'-methyl]butyl-5-methyl-(7,7)-dimethyl-octanoyl or undec-2-enoyl.

Exemplarily, said compounds may have structures of the following formulae. The structural formulae of fulvestrant esters I-XI are shown below:

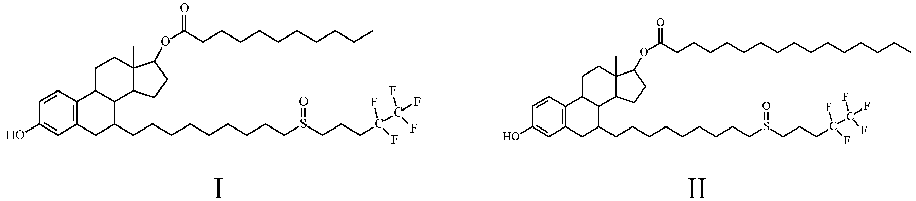

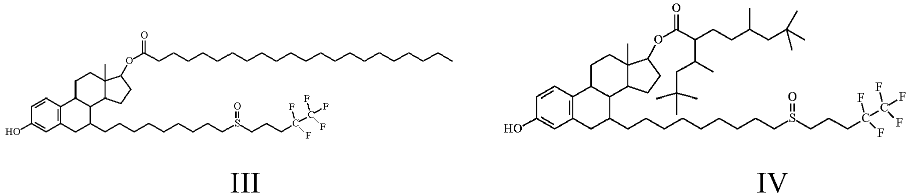

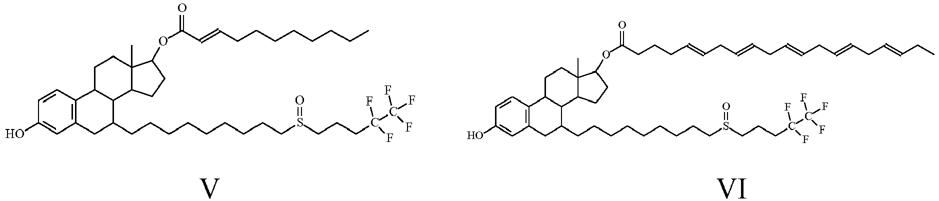

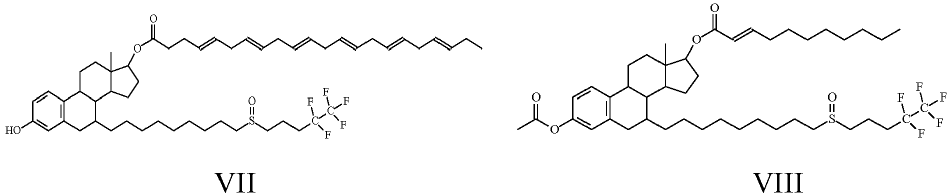

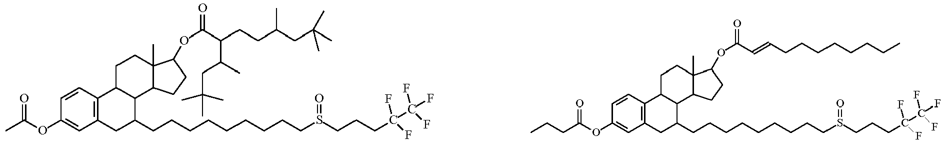

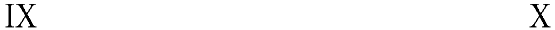

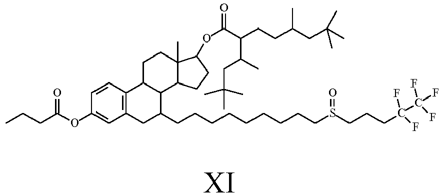

Furthermore, the present invention provides a process for preparing the compounds described as above, said process comprises the steps of:
a) acylating the -OH at C-17 position of compound of formula B: a compound of formula B is mixed with an alkaline reagent, an organic acid and N,N-dicyclohexylcarbodiimide in a solvent at room temperature under stirring to form a reaction mixture, said reaction mixture is reacted to obtain a crude product of compound of Formula A with C-17 position acylated; 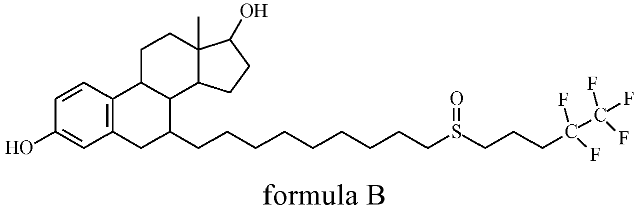
b) purifying the crude product obtained in step a) to remove the by-product N,N-dicycloalkylurea and obtain a purified product of compound of Formula A with C-17 position acylated;
when said substituent R' in the compounds is not H, said process further comprises the steps of:
c) acylating C-3 position of the purified product with C-17 position acylated obtained in step b): the purified product with C-17 position acylated obtained in step b) is mixed with an alkaline reagent, an organic acid and a catalyst in a solvent at room temperature under stirring to be reacted to obtain a crude product of compound of Formula A with C-17 and C-3 positions acylated;
d) purifying the crude product obtained in step c) to obtain a purified product of compound of Formula A.

Wherein, in step a), said alkaline reagent is selected from pyridine, 2-methylpyridine, 3-methylpyridine, 4-methylpyridine, 2-ethylpyridine, 3-ethylpyridine, 4-ethylpyridine, 5-ethylpyridine, 2-methyl-5-ethylpyridine, 2-dimethylaminopyridine, 4-dimethylaminopyridine, preferably 4-dimethylaminopyridine; said solvent is selected from methyl chloride, methylene chloride, chloroform;

N,N-dicyclohexylcarbodiimide; said organic acid is alkyl acid or alkenyl acid having 2 to 22 carbon atoms; in step b), said purifying comprises the step of dissolving the crude product obtained in step a) in tetrahydrofuran or ethyl acetate to form a solution, then settling the solution with n-hexane or mixed solvent of n-hexane-ethyl acetate, separating and purifying the settled solution by silica-gel column chromatography and/or neutral alumina adsorption; in step c), said alkaline reagent is selected from pyridine, 2-methylpyridine, 3-methylpyridine, 4-methylpyridine, 2-ethylpyridine, 3-ethylpyridine, 4-ethylpyridine, 5-ethylpyridine, 2-methyl-5-ethylpyridine, 2-dimethylaminopyridine, 4-dimethylaminopyridine, preferably 4-dimethylaminopyridine; said solvent is selected from tetrahydrofuran, ethyl acetate, preferably tetrahydrofuran; said catalyst is dehydrating agent, preferably N,N-dicyclohexylcarbodiimide; said organic acid is alkyl acid or alkenyl acid having 2 to 4 carbon atoms; in step d), said purifying is carried out by silica-gel column chromatography and ethanol elution, wherein mixed solvent of n-hexane-ethyl acetate is used for gradient elution in said silica-gel column chromatography, the volume ratio of n-hexane to ethyl acetate is 50:1-1:1, preferably 40:1 / 10:1 / 5:1 for gradient elution.

Furthermore, the present invention provides a composition comprising a compound of Formula A described as above, wherein, said composition is an oil, a fat or a microsphere agent.

Furthermore, the present invention also provides a compound of Formula A described as above or a composition comprising a compound of Formula A for use in the treatment of cancer; said medicament is preferably for use in inhibiting cancer cells with estrogen receptors, particularly preferably for use in inhibiting breast cancer cells.

Furthermore, the present invention also provides the use of a compound of Formula A described as above or a composition comprising a compound of Formula A for the manufacture of a medicament in the treatment of cancer; said medicament is preferably for use in inhibiting cancer cells with estrogen receptors, particularly preferably for use in inhibiting breast cancer cells.

Exemplarily, after being formulated into oiling agent, the compound(s) according to the present invention is administered to nude mice bearing human breast cancer MCF-7 tumor by subcutaneous injection to study the tumor inhibition rate. The result showed that such derivatives have anticancer activity for treating breast cancer.

### Description of the Preferred Embodiments

Hereinafter, the present invention will be further described in detail in combination with specific embodiments. The examples given are only for illustration, but not for limiting the scope of the present invention.

### Synthesis Examples

Although the alkaline reagent in the examples below is 4-dimethylaminopyridine, it is understood that agents such as pyridine, 2-methylpyridine, 3-methylpyridine, 4-methylpyridine, 2-ethylpyridine, 3-ethylpyridine, 4-ethylpyridine, 5-ethylpyridine, 2-methyl-5-ethylpyridine, 2-dimethylaminopyridine and the like can also be used in the examples below as alkaline reagents.

### Example 1 Synthesis and structure confirmation of Compound II

### 1) Reaction treatment

5g (8.25mmol) fulvestrant was added into a 500mL three-necked round-bottom flask and dissolved with 300mL methylene chloride while stirring. Then, 0.137g (1.1mmol) 4-dimethylaminopyridine (DMAP), 2.155g (8.41mmol) palmitic acid and 1.672g (8.23mmol) N,N-dicyclohexylcarbodiimide (DCC) was added sequentially into said flask. After reacting at room temperature (e.g., 20±5°C) for 48h, the reaction was stopped.

### 2) Post process

The reaction mixture was filtered to remove precipitated by-product N,N'-dicyclohexylurea (DCU). The filtrate was washed with saturated sodium bicarbonate solution, then washed with water to neutral, and then evaporated by rotary evaporator to remove methylene chloride. Colorless and clear colloidal liquid (8.8g) was obtained, which was dissolved in an appropriate amount of ethyl acetate, froze in a refrigerator (e.g., the freezing temperature may be -15±3°C). A small amount of white solid precipitated was washed out and removed by filtration for 3 times. Then, the filtrate was evaporated by rotary evaporator to remove ethyl acetate, and colorless and clear colloidal liquid was obtained. The colorless and clear colloidal liquid was dissolved in a small amount of tetrahydrofuran, then the solution was added to n-hexane to form a large quantity of white solid, which was left to stand and filtrated; the filter cake was dissolved in the aforesaid tetrahydrofuran and settled in n-hexane for 3 times to give white powder product, which was pure Compound II. The product was dried in vacuum at 60°C to give 1.5g II (purity 99.88% as determined by HPLC, C18 column, mobile phase: 67% THF in water, flow rate: 1.0 mL/min, detection wavelength: 220nm), and the molar yield was 22%.
IR(cm⁻¹): 3209, 2922, 2852, 1607, 1503, 1446, 1385, 1106, 1055, 1014, 982.
¹HNMR(500MHz, CDCl3, ppm): 0.78(s, 3H), 0.88(t, 3H), 1.01-1.52(t, 32H), 1.59-1.63(t, 6H), 1.70-1.76(t, 6H), 1.89-1.94(t, 2H), 2.10-2.32(t, 10H), 2.61-2.85(t, 8H), 3.74(t, 2H), 6.20(d, j=10 Hz, 1H), 6.56-7.14(t, 3H).
¹³CNMR(125MHz, CDCl3, ppm): 172.67, 154.23, 136.88, 131.04, 126.93, 117.67, 113.01, 82.02, 52.41, 50.83, 46.49, 43.40, 42.05, 38.23, 36.92, 34.74, 34.65, 33.35, 33.24, 31.93, 30.51, 29.92-28.22, 27.24, 25.62, 25.00, 22.63, 14.65, 14.09, 11.12.

### Example 2 Synthesis and structure confirmation of Compound I

### 1) Reaction treatment

3g (4.95mmol) fulvestrant was added into a 250mL round-bottom flask and dissolved with 160mL methylene chloride while stirring. Then 0.0822g (0.66mmol) DMAP, 0.96g (5.05mmol) undecanoic acid and 1.02g (4.98mmol) DCC was added sequentially into said flask. After reacting under stirring at room temperature (e.g., 20±5°C) for 48h, the reaction was stopped.

### 2) Post process

The reaction system was first frozen to precipitate as much reaction by-product DCU as possible. After being filtered to remove solid DCU, the filtrate was washed with saturated sodium bicarbonate solution, then washed with water to neutral and evaporated by rotary evaporator to remove methylene chloride, to give colorless and clear colloidal liquid, which was dissolved in a small amount of ethyl acetate and then froze in a refrigerator (e.g., the freezing temperature may be -15±3°C) until no white solid DCU precipitated out. The filtrate was concentrated to remove ethyl acetate, recrystallized from mixed solvent of n-hexane-ethyl acetate, and then filtered to remove white solid precipitated out (unreacted raw material fulvestrant). The mother liquor was spin-dried to give colorless oily matter. Said oily matter was further purified by silica-gel column chromatography (the eluent was n-hexane-ethyl acetate (1:1, volume ratio)) and was then evaporated by rotary evaporator to give 1.0611g colorless oily matter, which was Compound I (purity 99.104% as determined by HPLC according to the same determination method in Example 1), and the molar yield was 27.7%.
IR(cm-1): 3385, 2926, 2855, 1756, 1494, 1463, 1199, 1152, 1059, 1017, 985, 721.
¹HNMR(500 MHz, CDCl3, ppm): δ 7.28 (s, 1 H), 6.83 (d, 1 H), 6.77 (d, 1 H), 3.73 (t, 1 H, J=8 Hz), 2.88-1.17 (t, 57 H), 0.89 (s, 3 H), 0.77 (s, 3 H).
¹³CNMR(125 MHz, CDCl3, ppm): δ 172.64, 148.52, 137.13, 126.91, 122.37, 120.10, 118.64, 81.93, 52.75, 51.03, 46.47, 43.33, 41.67, 38.23, 36.89, 34.50, 34.45, 33.85, 31.89, 29.67, 29.50, 29.63, 29.55, 29.49, 29.46, 29.34, 29.30, 29.26, 29.16, 29.12, 28.80, 28.23, 27.11, 25.70, 25.01, 24.88, 22.66, 14.62, 14.50, 11.50.

### Example 3 Synthesis and structure confirmation of Compound III

3g (4.95mmol) fulvestrant was added into a 250mL round-bottom flask and then dissolved with 160mL methylene chloride while stirring. Then, 0.0822g (0.66mmol) DMAP, 1.87g (5.05mmol) docosanoic acid and 1.02g (4.98mmol) DCC was added sequentially into said flask. After reacting under stirring at room temperature (e.g., 20±5°C) for 48h, the reaction was stopped.

Reaction liquid was treated according to the post process in Example 2 to give 1.016g white solid powder (purity 92.634%, determined by HPLC) (C18 column, mobile phase: 75% THF in water, flow rate: 1.0 mL/min, detection wavelength: 220nm), which was Compound III, and the molar yield was 22.1%.
IR(cm-1): 3607, 3424, 2919, 2851, 1754, 1495, 1471, 1199, 1153, 1141, 1112, 1081,985,719.
¹HNMR(500 MHz, CDCl3, ppm): δ 7.28 (d, 1 H), 6.83 (d, 1 H), 6.77 (d, 1 H), 3.74 (t, 1 H, J=8 Hz), 2.91-1.05 (t, 79 H), 0.89 (t, 3 H), 0.77 (s, 3 H).
¹³CNMR(125 MHz, CDCl3, ppm): δ 172.64, 148.53, 137.13, 126.91, 122.37, 118.64, 81.93, 52.83, 51.11, 46.48, 43.34, 41.68, 38.24, 36.89, 34.50, 33.15, 31.94, 30.56, 29.94, 29.86, 29.71, 29.67, 29.63, 29.62, 29.51, 29.48, 29.37, 29.35, 29.27, 29.17, 29.13, 28.81, 28.23, 27.12, 25.70, 25.01, 24.88, 23.16, 22.66, 14.50, 14.01, 11.50.

### Example 4 Synthesis and structure confirmation of Compound IV

3g (4.95 mmol) fulvestrant was added into a 250mL round-bottom flask and then dissolved with 160mL methylene chloride while stirring. Then, 0.0822g (0.66mmol) DMAP, 1.44g (5.05mmol) isostearic acid and 1.02g (4.98mmol) DCC was added sequentially into said flask. After reacting under stirring at room temperature (e.g., 20±5°C) for 48h, the reaction was stopped.

Reaction liquid was treated according to the post process in Example 2 to give 1.0028g colorless colloid (purity 99.312%, determined by HPLC) (according to the same determination method in Example 3), which was Compound IV, and the molar yield was 23.2%.
IR(cm-1): 3396, 2928, 2866, 1748, 1494, 1466, 1364, 1198, 1149, 1121, 1058, 1017, 984, 720.
¹HNMR(500 MHz, CDCl3, ppm): δ7.28 (s, 1 H), 6.83 (d, 1 H), 6.76 (s, 1 H), 3.74 (t, 1 H, J=8 Hz), 2.35-1.03 (t, 71 H), 1.09-0.94 (t, 3 H), 0.89 (s, 3 H), 0.77 (s, 3 H).
¹³CNMR (125 MHz, CDCl3, ppm): δ 171.15, 148.60, 137.03, 126.88, 122.45, 118.72, 81.94, 53.34, 53.04, 52.82, 51.39, 50.96, 48.46, 48.39, 48.32, 46.48, 43.33, 41.68, 38.21, 37.92, 37.86, 37.79, 36.89, 34.50, 33.16, 32.37, 32.05, 31.11, 30.56, 30.06, 29.96, 29.87, 29.69, 29.55, 29.50, 29.37, 29.32, 29.18, 28.81, 28.26, 27.11, 26.09, 25.65, 24.81, 22.66, 21.21, 19.40, 14.61, 14.50, 11.51.

### Example 5 Synthesis and structure confirmation of Compound V

0.36g (0.6 mmol) fulvestrant was added into a 50mL round-bottom flask and then dissolved with 25 mL methylene chloride while stirring. Then, 9.93mg (0.08mmol) DMAP, 0.113g (0.61mmol) undecenoic acid and 0.13g (0.64 mmol) DCC was added sequentially into said flask. After reacting under stirring at room temperature (e.g., 20±5°C) for 48h, the reaction was stopped.

Reaction liquid was treated according to the post process in Example 2 to give light yellow oily matter, which was further purified by silica-gel column chromatography for 3 times and neutral alumina for once and was evaporated to dryness to give 0.1g light yellow oily matter (purity 96.010%, determined by HPLC) (according to the same determination method in Example 3). The obtained light yellow oily matter was Compound V, and the molar yield was 21.5%.
IR(KBr, cm-1): 3387, 2927, 2855, 1736, 1652, 1494, 1461, 1356, 1312, 1198, 1154, 1121, 1059, 1016, 983, 721.
¹HNMR(500 MHz, CDCl3, ppm): δ 7.27 (t, 1 H), 7.15 (t, 1 H), 6.87 (s, 1 H), 6.82 (s, 1 H), 6.43 (t, 2 H), 5.99 (t, 1 H), 3.74 (t, 1 H, J=8 Hz), 3.2-1.1 (t, 51 H), 0.89 (t, 3 H, J=7 Hz), 0.77 (s, 3 H).
¹³CNMR(125 MHz, CDCl3, ppm): δ 170.90, 165.38, 151.71, 148.55, 137.10, 135.55, 126.91, 122.44, 120.94, 120.12, 118.79, 81.93, 52.77, 51.04, 46.50, 43.35, 41.71, 38.27, 36.91, 34.51, 33.18, 31.85, 30.56, 29.94, 29.87, 29.70, 29.62, 29.51, 29.36, 29.34, 29.19, 29.16, 29.09, 28.96, 28.81, 28.23, 27.13, 25.70, 24.88, 22.66, 14.50, 13.50, 11.10.

### Example 6 Synthesis and structure confirmation of Compound VI

0.36g (0.6 mmol) fulvestrant was added into a 50mL round-bottom flask and then, dissolved with 25 mL methylene chloride while stirring. Then, 9.93mg (0.08mmol) DMAP, 0.185g (0.61mmol) eicosapentaenoic acid and 0.13g (0.64 mmol) DCC was added sequentially into said flask. After reacting under stirring at room temperature (e.g., 20±5°C) for 48h, the reaction was stopped.

Reaction liquid was treated according to the post process in Example 2 to give 0.31mg light yellow oily matter (purity 99.195%, determined by HPLC with the method referred to the method in Example 3), which was Compound VI, and the yield was 58%.
IR(cm-1): 3396, 3012, 2927, 2855, 1756, 1609, 1494, 1456, 1312, 1198, 1137, 1058, 1018, 985, 719.
¹HNMR(500 MHz, CDCl3, ppm): δ 7.28 (t, 1 H), 6.84 (t, 1 H, J=7.5 Hz), 6.77 (d, 1 H), 5.43-5.32 (t, 10 H), 3.74 (t, 1 H, J=8 Hz), 2.87-1.18 (t, 55 H), 0.97 (t, 3 H, J=7.5 Hz), 0.77 (s, 3 H).
¹³CNMR(125 MHz, CDCl3, ppm): δ 172.38, 137.19, 132.05, 129.07, 128.84, 128.59, 128.29, 128.22, 128.10, 127.89, 127.03, 126.94, 122.34, 118.62, 81.94, 52.76, 51.05, 46.48, 43.34, 41.67, 38.23, 36.89, 34.50, 33.78, 33.15, 30.56, 29.95, 29.86, 29.68, 29.65, 29.50, 29.36, 29.18, 28.82, 28.24, 27.12, 26.56, 25.70, 25.66, 25.65, 25.56, 24.82, 22.66, 20.85, 14.50, 13.50, 11.50.

### Example 7 Synthesis and structure confirmation of Compound VII

0.36g (0.6 mmol) fulvestrant was added into a 50mL round-bottom flask and then dissolved with 25 mL methylene chloride while stirring. Then, 9.93mg (0.08mmol) DMAP, 0.2g (0.61mmol) docosahexenoic acid and 0.13g (0.64 mmol) DCC was added sequentially into said flask. After reacting under stirring at room temperature (e.g., 20±5°C) for 48h, the reaction was stopped.

Reaction liquid was treated according to the post process in Example 2 to give 0.1165g light yellow oily matter (purity 99.051%, determined by HPLC with the method referred to the method in Example 3), which was Compound VII, and the yield was 21.1%.
IR(cm-1): 3396, 3013, 2927, 2855, 1756, 1609, 1494, 1456, 1358, 1198, 1138, 1059, 1018, 984, 719.
¹HNMR(500 MHz, CDCl3, ppm): δ 7.27 (t, 1 H), 6.83 (d, 1 H), 6.77 (t, 1 H), 5.4-5.3 (t, 12 H), 3.74 (t, J=8 Hz, 1 H), 2.8-1.1 (t, 55 H), 0.97 (t, 3 H), 0.77 (s, 3 H).
¹³CNMR(125 MHz, CDCl3, ppm): δ 171.88, 148.47, 137.22, 132.05, 129.62, 128.58, 128.33, 128.29, 128.26, 128.11, 128.09, 128.04, 127.89, 127.64, 127.03, 126.93, 122.35, 118.62, 81.94, 52.76, 51.05, 46.48, 43.34, 41.67, 38.23, 36.89, 34.50, 34.34, 33.14, 30.56, 29.94, 29.85, 29.68, 29.65, 29.50, 29.36, 29.18, 28.82, 28.24, 27.12, 25.70, 25.66, 25.64, 25.55, 22.85, 22.66, 22.58, 20.57, 14.30, 14.10, 11.50.

### Example 8 Synthesis and structure confirmation of Compound VIII

### 1) Reaction treatment

0.31 g (0.4 mmol) Compound V (synthesized in Example 5), 4 mL (40 mmol) acetic anhydride, 0.2 g (1.6 mmol) 4-dimethylaminopyridine (DMAP) was sequentially added into a 50mL round-bottom flask. After reflux reacting for 48 h, the reaction was stopped.

### 2) Post process

After the reaction system was cooled, it was washed with water to neutral and phase separated. The organic layer was spin-dried and purified by silica-gel column chromatography through gradient eluting (the eluent was n-hexane-ethyl acetate (40:1 / 10:1 / 5:1, volume ratio)). Then, the eluent was evaporated to dryness to give milky white colloidal liquid, which was Compound VIII.
IR(cm-1): 3449, 2927, 2855, 1736, 1651, 1494, 1461, 1373, 1360, 1311, 1245, 1198, 1154, 1121, 1045, 1027, 983, 896, 822, 720.
¹HNMR(500 MHz, CDCl3, ppm): δ 7.27 (t, 1 H), 7.15(t, 1 H), 6.87(t, 1 H), 6.81 (d, 1 H), 6.40 (t, 1 H), 6.00 (d, 1 H), 5.63 (t, 1 H), 4.70 (t, 1 H), 2.7-1.1 (t, 52 H), 2.05 (t, 3 H), 0.89 (t, 3 H), 0.82 (s, 3 H).
¹³CNMR(125 MHz, CDCl3, ppm): δ 170.90, 165.36, 151.72, 148.56, 137.07, 136.97, 126.92, 122.43, 122.32, 120.92, 118.73, 82.76, 52.71, 50.98, 46.26, 42.94, 41.40, 38.20, 38.12, 37.06, 34.50, 33.17, 32.50, 32.41, 31.84, 29.85, 29.67, 29.55, 29.49, 29.35, 29.32, 29.18, 29.15, 28.79, 28.16, 26.96, 25.64, 22.78, 22.65, 21.17, 14.63, 12.02.

### Example 9 Synthesis and structure confirmation of Compound IX

0.3 g (0.35mmol) Compound IV (synthesized in Example 4), 3.5 mL (35mmol) acetic anhydride and 0.18 g (1.44 mmol) 4-dimethylaminopyridine (DMAP) was sequentially added and then 30mL tetrahydrofuran was added into a 50mL round-bottom flask. After reflux reacting for 48 h, the reaction was stopped.

Reaction liquid was treated according to the post process in Example 8 to give milky white colloidal liquid, which was Compound IX.
IR(cm-1): 3311, 2927, 2854, 1736, 1665, 1494, 1460, 1365, 1245, 1200, 1045, 1027, 984, 803, 720.
¹HNMR(500 MHz, CDCl3, ppm): δ 7.27 (t, 1 H), 6.82 (t, 1 H), 6.76 (t, 1 H), 4.70 (t, 1 H), 2.77-1.08 (t, 49 H), 2.05 (t, 3H), 0.81-0.95 (t, 27 H).
¹³CNMR(125 MHz, CDCl3, ppm): δ 174.36, 171.23, 148.55, 136.95, 126.90, 122.45, 122.39, 118.74, 118.71, 82.76, 53.11, 53.03, 52.81, 51.39, 48.45, 48.31, 46.25, 42.94, 41.40, 38.07, 37.78, 37.05, 34.50, 33.16, 32.36, 32.05, 31.93, 31.44, 30.19, 30.05, 30.03, 29.88, 29.67, 29.55, 29.49, 29.35, 29.30, 29.17, 28.80, 28.19, 27.52, 26.99, 25.66, 22.69, 21.17, 20.35, 19.93, 14.63, 12.02.

### Example 10 Synthesis and structure confirmation of Compound X

0.3 g (0.35mmol) Compound V (synthesized in Example 5), 3.5 mL (35mmol) butyric anhydride and 0.18 g (1.44 mmol) 4-dimethylaminopyridine (DMAP) was sequentially added and then 30mL tetrahydrofuran was added into a 50mL round-bottom flask. After reflux reacting for 48 h, the reaction was stopped.

Reaction liquid was treated according to the post process in Example 8 to give milky white colloidal liquid, which was Compound X.
IR(cm-1): 3441, 2927, 2855, 1734, 1651, 1494, 1460, 1197, 1154, 1120, 1092, 1019, 983, 803, 720.
¹HNMR(500 MHz, CDCl3, ppm): δ 7.27 (t, 1 H), 7.15 (t, 1 H), 6.88 (t, 1 H), 6.81 (d, 1 H), 6.40 (t, 1 H), 6.00 (d, 1 H), 5.63 (t, 1 H), 4.70 (t, 1 H), 2.7-1.0 (t, 56 H), 0.96 (t, 3 H), 0.88 (t, 3H), 0.82 (t, 3H).
¹³CNMR(125 MHz, CDCl3, ppm): δ 173.79, 165.38, 151.73, 148.57, 137.02, 136.99, 126.93, 122.45, 122.39, 120.60, 118.67, 82.45, 52.73, 50.99, 46.28, 43.02, 41.42, 38.21, 38.14, 37.10, 36.52, 36.28, 34.51, 33.19, 32.51, 32.43, 31.85, 29.86, 29.68, 29.56, 29.50, 29.36, 29.33, 29.19, 29.17, 28.81, 28.18, 27.58, 26.99, 25.65, 22.82, 22.66, 18.69, 14.50, 12.02, 12.00.

### Example 11 Synthesis and structure confirmation of Compound XI

0.69g (0.89mmol) Compound IV (synthesized in Example 4), 14.5mL (89mmol) butyric anhydride and 0.44g (3.52mmol) 4-dimethylaminopyridine (DMAP) was sequentially added and then 69mL tetrahydrofuran was added into a 50mL round-bottom flask. After reflux reacting for 48 h, the reaction was stopped.

After the reaction system was cooled, it was washed with water to neutral and phase separated. The organic layer was spin-dried and purified by silica-gel column chromatography through gradient eluting (the eluent was n-hexane-ethyl acetate (40:1 / 10:1 / 5:1, volume ratio)). Then, the eluent was evaporated to dryness to give crude product, which was then treated by ultrasonic water washing for several times. During said water washing process, the crude product attached to walls of flask in the form of colloid and water phase was poured directly after washing. Washing was repeated until the product had no smell of butyric acid. Finally, the product was quickly eluted with ethanol and the solvent was removed in a decompressed oven to give milky white colloidal liquid, which was Compound XI.
IR(cm-1): 3448, 2390, 2857, 1750, 1734, 1609, 1494, 1465, 1364, 1198, 1150, 1121, 1094, 1048, 1019, 984, 905, 803, 732.
¹HNMR(500 MHz, CDCl3, ppm): δ 7.27 (t, 1 H), 6.82 (t, 1 H), 6.76 (s, 1 H), 4.71 (t, 1 H), 1.09-2.77 (t, 53 H), 0.81-1.08 (t, 30 H).
¹³CNMR(125 MHz, CDCl3, ppm): δ 174.36, 173.78, 148.54, 136.95, 126.90, 122.45, 122.39, 118.73, 118.71, 82.45, 53.33, 53.03, 52.82, 51.39, 48.45, 48.31, 46.27, 43.00, 41.41, 38.08, 37.76, 37.09, 36.52, 34.50, 33.17, 32.36, 32.05, 31.10, 31.07, 30.05, 30.03, 30.01, 29.88, 29.68, 29.59, 29.55, 29.50, 29.35, 29.31, 29.21, 29.17, 28.81, 28.20, 27.57, 27.01, 25.67, 22.58, 22.40, 19.93, 18.59, 14.64, 13.69, 12.06.

### Example of physicochemical properties

### Example 12 Solubility experiments of fulvestrant and ester derivatives thereof in different solvents

Fulvestrant and ester derivatives of fulvestrant were accurately weighed to an appropriate amount respectively. Their solubilities (in mg/mL) in different oils and solvents were compared according to General Notice in Section 2 of Chinese Pharmacopoeia (2010). The results are shown in Table 1:

**Table 1. Solubilities of fulvestrant and ester derivatives thereof in different oils and solvents**

| Solvent | Castor oil | Soybean oil | Medium-chain oil | PEG 400 | Propylene glycol |
|---|---|---|---|---|---|
| Compound II | >100.2 | >100 | >10 | 2.9 | 10.2 |
| Compound V | ND | 122 | ND | ND | 12.2 |
| Compound I | ND | 255 | ND | ND | 2.9 |
| CompoundIII | ND | 11 | ND | ND | 0.4 |
| Compound IV | ND | 28 | ND | ND | 7.1 |
| Fulvestrant | 20* | 5 | ND | 6.9 | 10.2 |

| | | | | | |
|---|---|---|---|---|---|
| Note: * denote the reported values. | | | | | |

It can be seen that, compared with the solubility of fulvestrant, the solubility of Compound II in lipophilic solvents including castor oil, soybean oil, medium-chain oil increased significantly, yet had almost no change in propylene glycol, and decreased significantly in hydrophilic solvent PEG 400; meanwhile, the solubilities of derivatives such as Compounds I, III and IV in lipophilic soybean oil were significantly greater than that of fulvestrant.

### Example of drug efficacy

### Example 13 The growth inhibition effects of fulvestrant, Compounds II and X on human breast cancer MCF-7 xenografted in nude mice

Test drugs: fulvestrant, Compounds II and X are dispersed in oil and sterilized to be prepared as oiling agents respectively.

Experimental animals and grouping thereof, source, germline and strain: BALB/c female nude mice, provided by Laboratory Animal Research Center of Academy of Military Medical Sciences of China (Laboratory animal production license: SCXK (Military) 2007-004), day-old: 35-40 days; body weight: 18-24g. The mice was divided into negative control group, positive control group (fulvestrant oiling agent), drug treatment groups (Compounds II and X oiling agent respectively), with 5 mice in each group.

Administration method, dose and time: the negative control group was administered with blank solvent (oil) by subcutaneous injecting 0.2mL/20g for once; positive control group was administered with fulvestrant oiling agent by subcutaneous injecting 100mg/kg for once; drug treatment groups were respectively administered with Compounds II and X by subcutaneous injecting 100mg/kg for once.

Establishment of model and tumor measuring method: human breast cancer MCF-7 cell lines in logarithmic growth phase were prepared into a cell suspension of 5 × 10⁸ /mL under sterile condition, with 0.1 mL of which being inoculated to nude mice at their right armpits subcutaneously. Xenografted tumors of nude mice were measured for diameter with vernier caliper, and animals were randomly grouped when the tumors grew to 100-300mm³. The administration volume to each of the mice was 0.2mL/20g by subcutaneous injection at head and neck region. 28 days after administration, the mice were sacrificed and the tumors were stripped by surgery and weighed. Tumor inhibition rate was calculated (inhibition rate = (1 - tumor weight in the experimental group/ tumor weight in the control group) × 100%). The results are shown in Table 2 below:

**Table 2. The growth inhibition effects of fulvestrant and ester derivatives thereof on human breast cancer MCF-7 xenografted in nude mice (X±SD)**

| Group | Dose (mg/kg) | Initial body weight(g) | Initial animal number | Final body weight (g) | Final animal number | Tumor weight (g) | Tumor inhibition rate (%) |
|---|---|---|---|---|---|---|---|
| Negative control group | - | 18.800±0.748 | 5 | 21.200±0.748 | 5 | 1.180±0.795 | - |
| Fulvestrant oiling agent | 100 | 18.400±0.490 | 5 | 15.800±1.166** | 5 | 0.392±0.443 | 66.78 |
| Compound II oiling agent | 100 | 18.400±0.800 | 5 | 15.200±0.748** | 5 | 0.426±0.306 | 64.90 |
| Compound X oiling agent | 100 | 18.800±0.748 | 5 | 15.600±1.020** | 5 | 0.402±0.711 | 65.93 |

Compared with the blank control group, *p<0.05, **p<0.01.

The results show that all of fulvestrant and ester derivatives thereof have anti breast cancer effects.

## Claims

1. A compound of Formula A: 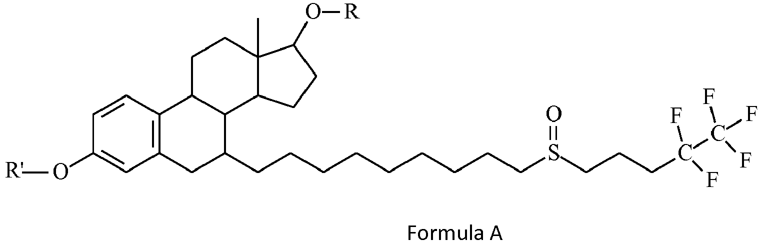 wherein,
substituent R' is selected from H, butyryl or alkenoyl having 2 to 4 carbon atoms,
substituent R is selected from alkanoyl or alkenoyl having 11 to 22 carbon atoms.

2. The compound of Claim 1, **characterized in that**,
said substituent R is selected from undecanoyl, hexadecanoyl, docosanoyl or 2-[(3',3')-dimethyl-l'-methyl]butyl-5-methyl-(7,7)-dimethyl-octanoyl.

3. The compound of Claim 1, **characterized in that**,
said substituent R is alkenoyl containing 1 to 6 carbon-carbon double bonds, wherein said carbon-carbon double bonds can either be distributed in the main chain, or in the branched chain.

4. The compound of Claim 3, **characterized in that**,
said substituent R is selected from undec-2-enoyl, eicosa-5,8,11,14,17- pentaenoyl and docosa-(4,7,10,13,16,19)-hexaenoyl.

5. The compound of Claim 1, **characterized in that**,
said substituent R is selected from 2-[(3',3')-dimethyl-1'-methyl]butyl-5-methyl-(7,7)-dimethyl-octanoyl or undec-2-enoyl.

6. A process for preparing a compound of any one of Claims 1 to 5, **characterized in that**, said process comprises the steps of:
a) acylating the -OH at C-17 position of compound of formula B: a compound of formula B is mixed with an alkaline reagent, an organic acid and N,N-dicyclohexylcarbodiimide in a solvent at room temperature under stirring to form a reaction mixture, said reaction mixture is reacted to obtain a crude product of compound of Formula A with C-17 position acylated; 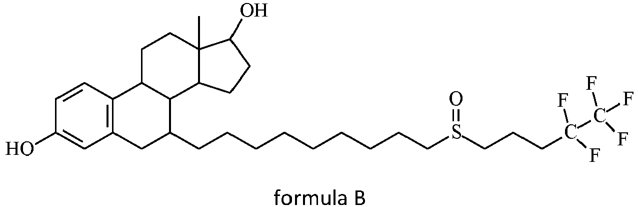
b) purifying the crude product obtained in step a) to remove the by-product N,N-dicycloalkylurea and obtain a purified product of compound of Formula A with C-17 position acylated;
when said substituent R' in the compound is not H, said process further comprises the steps of:
c) acylating C-3 position of the purified product with C-17 position acylated obtained in step b): the purified product with C-17 position acylated obtained in step b) is mixed with an alkaline reagent, an organic acid and a catalyst in a solvent at room temperature under stirring to be reacted to obtain a crude product of compound of Formula A with C-17 and C-3 positions acylated;
d) purifying the crude product obtained in step c) to obtain a purified product of compound of Formula A.

7. The process of Claim 6, **characterized in that**, in step a), said alkaline reagent is selected from pyridine, 2-methylpyridine, 3-methylpyridine, 4-methylpyridine, 2-ethylpyridine, 3-ethylpyridine, 4-ethylpyridine, 5-ethylpyridine, 2-methyl-5-ethylpyridine, 2-dimethylaminopyridine, 4-dimethylaminopyridine, preferably 4-dimethylaminopyridine; said solvent is selected from methyl chloride, methylene chloride, chloroform; said catalyst is N,N-dicyclohexylcarbodiimide; said organic acid is alkyl acid or alkenyl acid having 2 to 22 carbon atoms; in step b), said purifying comprises the step of dissolving the crude product obtained in step a) in tetrahydrofuran or ethyl acetate to form a solution, then settling the solution with n-hexane or mixed solvent of n-hexane-ethyl acetate, separating and purifying the settled solution by silica-gel column chromatography and/or neutral alumina adsorption; in step c), said alkaline reagent is selected from pyridine, 2-methylpyridine, 3-methylpyridine, 4-methylpyridine, 2-ethylpyridine, 3-ethylpyridine, 4-ethylpyridine, 5-ethylpyridine, 2-methyl-5-ethylpyridine, 2-dimethylaminopyridine, 4-dimethylaminopyridine, preferably 4-dimethylaminopyridine; said solvent is selected from tetrahydrofuran, ethyl acetate, preferably tetrahydrofuran; said catalyst is dehydrating agent, preferably N,N-dicyclohexylcarbodiimide; said organic acid is alkyl acid or alkenyl acid having 2 to 4 carbon atoms; in step d), said purifying is carried out by silica-gel column chromatography and ethanol elution, wherein mixed solvent of n-hexane-ethyl acetate is used for gradient elution in said silica-gel column chromatography, the volume ratio of n-hexane to ethyl acetate is 50:1-1:1, preferably 40:1 / 10:1 / 5:1 for gradient elution.

8. A composition comprising a compound of Formula A of any one of claims 1 to 5, **characterized in that**, said composition is an oil, a fat or a microsphere agent.

9. A composition comprising a compound of Formula A of any one of claims 1 to 5, or a composition of claim 8, for use in the treatment of cancer; said medicament is preferably for use in inhibiting cancer cells with estrogen receptors, particularly preferably for use in inhibiting breast cancer cells.

10. Use of a composition comprising a compound of Formula A of any one of claims 1 to 5 or a composition of claim 8 for the manufacture of a medicament in the treatment of cancer; said medicament is preferably for use in inhibiting cancer cells with estrogen receptors, particularly preferably for use in inhibiting breast cancer cells.

## Patentansprüche

1. Verbindung der Formel A: 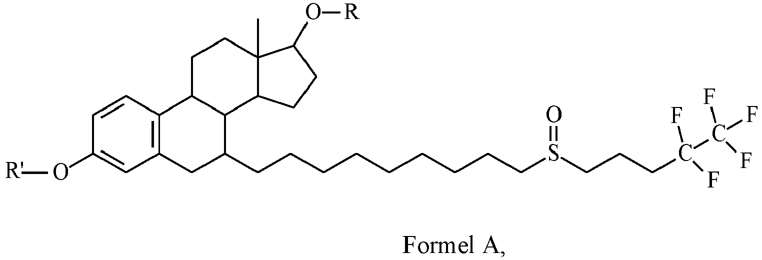 wobei Substituent R' ausgewählt ist aus H, Butyryl oder Alkenoyl mit 2 bis 4 Kohlenstoffatomen, Substituent R ausgewählt ist aus Alkanoyl oder Alkenoyl mit 11 bis 22 Kohlenstoffatomen.

2. Verbindung nach Anspruch 1, **dadurch gekennzeichnet, dass** Substituent R ausgewählt ist aus Undecanoyl, Hexadecanoyl, Docosanoyl oder 2-[(3',3')-Dimethyl-1'-methyl]butyl-5-methyl-(7,7)-dimethyl-octanoyl.

3. Verbindung nach Anspruch 1, **dadurch gekennzeichnet, dass** Substituent R Alkenoyl ist, das 1 bis 6 Kohlenstoff-Kohlenstoff-Doppelbindungen enthält, wobei die Kohlenstoff-Kohlenstoff-Doppelbindungen entweder in der Hauptkette oder in der verzweigten Kette verteilt sein können.

4. Verbindung nach Anspruch 3, **dadurch gekennzeichnet, dass** Substituent R ausgewählt ist aus Undec-2-enoyl, Eicosa-5,8,11,14,17-pentaenoyl und Docosa-(4,7,10,13,16,19)-hexaenoyl.

5. Verbindung nach Anspruch 1, **dadurch gekennzeichnet, dass** Substituent R ausgewählt ist aus 2-[(3',3')-Dimethyl-1'-methyl]butyl-5-methyl-(7,7)-dimethyl-octanoyl oder Undec-2-enoyl.

6. Verfahren zum Herstellen einer Verbindung nach einem der Ansprüche 1 bis 5, **dadurch gekennzeichnet, dass** das Verfahren die folgenden Schritte umfasst:
a) Acylieren des -OH an der Position C-17 der Verbindung der Formel B: eine Verbindung der Formel B wird mit einem alkalischen Reagens, einer organischen Säure und N,N-Dicyclohexylcarbodiimid in einem Lösungsmittel bei Raumtemperatur unter Rühren gemischt, um ein Reaktionsgemisch zu bilden, wobei das Reaktionsgemisch umgesetzt wird, um ein Rohprodukt der Verbindung der Formel A mit acylierter Position C-17 zu erlangen; 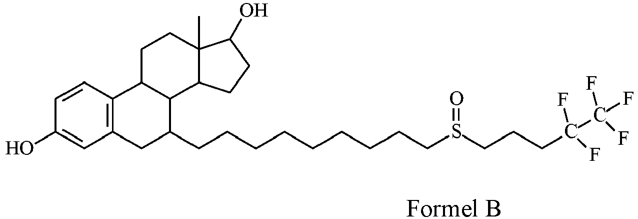
b) Aufreinigen des in Schritt a) erlangten Rohprodukts, um das Nebenprodukt N,N-Dicycloalkylharnstoff zu entfernen und ein aufgereinigtes Produkt der Verbindung der Formel A mit acylierter Position C-17 zu erlangen;
wenn Substituent R 'in der Verbindung nicht H ist, umfasst das Verfahren ferner die folgenden Schritte:
c) Acylieren der Position C-3 des in Schritt b) erlangten aufgereinigten Produkts mit acylierter Position C-17: das in Schritt b) erlangte aufgereinigte Produkt mit acylierter Position C-17 wird mit einem alkalischen Reagens, einer organischen Säure und einem Katalysator in einem Lösungsmittel bei Raumtemperatur unter Rühren gemischt, um zum Erlangen eines Rohprodukts der Verbindung der Formel A mit acylierter Position C-17 und C-3 umgesetzt zu werden;
d) Aufreinigen des in Schritt c) erlangten Rohprodukts, um ein aufgereinigtes Produkt der Verbindung der Formel A zu erlangen.

7. Verfahren nach Anspruch 6, **dadurch gekennzeichnet, dass** in Schritt a) das alkalische Reagens ausgewählt ist aus Pyridin, 2-Methylpyridin, 3-Methylpyridin, 4-Methylpyridin, 2-Ethylpyridin, 3-Ethylpyridin, 4-Ethylpyridin, 5-Ethylpyridin 2-Methyl-5-ethylpyridin, 2-Dimethylaminopyridin, 4-Dimethylaminopyridin, vorzugsweise 4-Dimethylaminopyridin; das Lösungsmittel ausgewählt ist aus Methylchlorid, Methylenchlorid, Chloroform; der Katalysator N,N-Dicyclohexylcarbodiimid ist; die organische Säure Alkylsäure oder Alkenylsäure mit 2 bis 22 Kohlenstoffatomen ist; in Schritt b) das Aufreinigen den Schritt eines Lösens des in Schritt a) erlangten Rohprodukts in Tetrahydrofuran oder Ethylacetat umfasst, um eine Lösung zu bilden, dann Absetzen der Lösung mit n-Hexan oder einem Lösungsmittelgemisch aus n-Hexan-Ethylacetat, Trennen und Aufreinigen der abgesetzten Lösung durch Kieselgel-Säulenchromatographie und/oder Adsorption von neutralem Aluminiumoxid; in Schritt c) das alkalische Reagens ausgewählt ist aus Pyridin, 2-Methylpyridin, 3-Methylpyridin, 4-Methylpyridin, 2-Ethylpyridin, 3-Ethylpyridin, 4-Ethylpyridin, 5-Ethylpyridin, 2-Methyl-5-ethylpyridin, 2-Dimethylaminopyridin, 4-Dimethylaminopyridin, vorzugsweise 4-Dimethylaminopyridin; das Lösungsmittel ausgewählt ist aus Tetrahydrofuran, Ethylacetat, vorzugsweise Tetrahydrofuran; der Katalysator ein Dehydratisierungsmittel ist, vorzugsweise N,N-Dicyclohexylcarbodiimid; die organische Säure Alkylsäure oder Alkenylsäure mit 2 bis 4 Kohlenstoffatomen ist; in Schritt d) die Aufreinigung durch Kieselgel-Säulenchromatographie und Ethanol-Elution durchgeführt wird, wobei zur Gradientenelution bei der Kieselgel-Säulenchromatographie ein Lösungsmittelgemisch aus n-Hexan-Ethylacetat verwendet wird, das Volumenverhältnis von n-Hexan zu Ethylacetat 50:1-1: 1, vorzugsweise 40:1/10:1/5:1 für die Gradientenelution beträgt.

8. Zusammensetzung, umfassend eine Verbindung der Formel A nach einem der Ansprüche 1 bis 5, **dadurch gekennzeichnet, dass** die Zusammensetzung ein Öl, ein Fett oder ein Mikrokugelmittel ist.

9. Zusammensetzung, umfassend eine Verbindung der Formel A nach einem der Ansprüche 1 bis 5 oder eine Zusammensetzung nach Anspruch 8 zur Verwendung bei der Behandlung von Krebs; wobei das Medikament bevorzugt zum Hemmen von Krebszellen mit Östrogenrezeptoren verwendet wird, besonders bevorzugt zum Hemmen von Brustkrebszellen verwendet wird.

10. Verwendung einer Zusammensetzung, umfassend eine Verbindung der Formel A nach einem der Ansprüche 1 bis 5 oder eine Zusammensetzung nach Anspruch 8 zum Herstellen eines Medikaments bei der Behandlung von Krebs; wobei das Medikament bevorzugt zum Hemmen von Krebszellen mit Östrogenrezeptoren verwendet wird, besonders bevorzugt zum Hemmen von Brustkrebszellen verwendet wird.

## Revendications

1. Composé de Formule A : 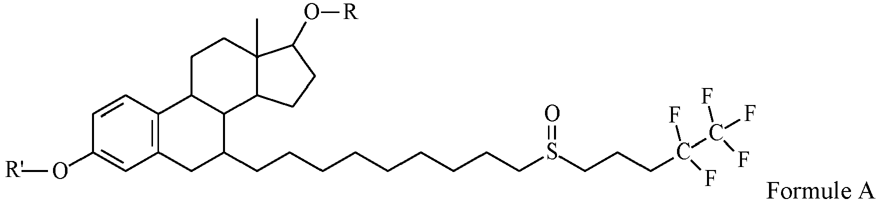 dans laquelle, le substituant R' est choisi parmi H, butyryle ou un alcénoyle ayant 2 à 4 atomes de carbone, le substituant R est choisi parmi un alcanoyle ou un alcénoyle ayant 11 à 22 atomes de carbone.

2. Composé selon la revendication 1, **caractérisé en ce que**, ledit substituant R est choisi parmi l'undécanoyle, l'hexadécanoyle, le docosanoyle ou le 2-[(3',3')-diméthyl-1'-méthyl]butyl-5-méthyl-(7,7)-diméthyl-octanoyle.

3. Composé selon la revendication 1, **caractérisé en ce que**, ledit substituant R est un alcénoyle contenant 1 à 6 doubles liaisons carbone-carbone, dans lequel lesdites doubles liaisons carbone-carbone peuvent être distribuées soit dans la chaîne principale, soit dans la chaîne ramifiée.

4. Composé selon la revendication 3, **caractérisé en ce que**, ledit substituant R est choisi parmi l'undéc-2-énoyle, l'éicosa-5,8,11,14,17-pentaénoyle et le docosa-(4,7,10,13,16,19)-hexaénoyle.

5. Composé selon la revendication 1, **caractérisé en ce que**, ledit substituant R est choisi parmi le 2-[(3',3')-diméthyl-1'-méthyl]butyl-5-méthyl-(7,7)-diméthyl-octanoyle ou l'undéc-2-énoyle.

6. Procédé de préparation d'un composé selon l'une quelconque des revendications 1 à 5, **caractérisé en ce que**, ledit procédé comprend les étapes de :
a) acylation de -OH au niveau de la position C-17 du composé de Formule B : un composé de Formule B est mélangé avec un réactif alcalin, un acide organique et le N,N-dicyclohexylcarbodiimide dans un solvant à température ambiante sous agitation afin de former un mélange réactionnel, ledit mélange réactionnel est mis à réagir afin d'obtenir un produit brut du composé de Formule A comportant la position C-17 acylée ; 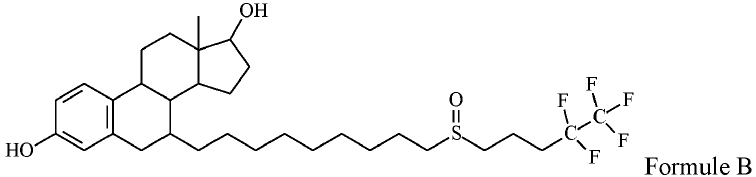
b) purification du produit brut obtenu lors de l'étape a) afin d'éliminer le produit secondaire N,N-dicycloalkylurée et pour obtenir un produit purifié du composé de Formule A comportant la position C-17 acylée ;
lorsque ledit substituant R' dans le composé n'est pas H, ledit procédé comprend en outre les étapes de :
c) acylation de la position C-3 du produit purifié comportant la position C-17 acylée obtenue lors de l'étape b) : le produit purifié comportant la position C-17 acylée obtenue lors de l'étape b) est mélangé avec un réactif alcalin, un acide organique et un catalyseur dans un solvant à température ambiante sous agitation pour être mise réagir afin d'obtenir un produit brut du composé de Formule A comportant les positions C-17 et C-3 acylées ;
d) purification du produit brut obtenu lors de l'étape c) afin d'obtenir un produit purifié du composé de Formule A.

7. Procédé selon la revendication 6, **caractérisé en ce que**, dans l'étape a), ledit réactif alcalin est choisi parmi la pyridine, la 2-méthylpyridine, la 3-méthylpyridine, la 4-méthylpyridine, la 2-éthylpyridine, la 3-éthylpyridine, la 4-éthylpyridine, la 5-éthylpyridine, la 2-méthyl-5-éthylpyridine, la 2-diméthylaminopyridine, la 4-diméthylaminopyridine, de préférence la 4-diméthylaminopyridine ; ledit solvant est choisi parmi le chlorure de méthyle, le chlorure de méthylène, le chloroforme ; ledit catalyseur est le N,N-dicyclohexylcarbodiimide ; ledit acide organique est un acide d'alkyle ou un acide d'alcényle ayant 2 à 22 atomes de carbone ; dans l'étape b), ladite purification comprend l'étape de dissolution du produit brut obtenu lors de l'étape a) dans le tétrahydrofurane ou l'acétate d'éthyle afin de former une solution, et ensuite le décantage de la solution avec le n-hexane ou un solvant mélangé de n-hexane-acétate d'éthyle, la séparation et la purification de la solution décantée par chromatographie sur colonne de gel de silice et/ou adsorption sur alumine neutre ; dans l'étape c), ledit réactif alcalin est choisi parmi la pyridine, la 2-méthylpyridine, la 3-méthylpyridine, la 4-méthylpyridine, la 2-éthylpyridine, la 3-éthylpyridine, la 4-éthylpyridine, la 5-éthylpyridine, la 2-méthyl-5-éthylpyridine, la 2-diméthylaminopyridine, la 4-diméthylaminopyridine, de préférence la 4-diméthylaminopyridine ; ledit solvant est choisi parmi le tétrahydrofurane, l'acétate d'éthyle, de préférence le tétrahydrofurane ; ledit catalyseur et un agent de déshydratation, de préférence le N,N-dicyclohexylcarbodiimide ; ledit acide organique est un acide d'alkyle ou un acide d'alcényle ayant 2 à 4 atomes de carbone ; dans l'étape d), ladite purification est effectuée par chromatographie sur colonne de gel de silice et élution par de l'éthanol, dans lequel le solvant mélangé de n-hexane-acétate d'éthyle est utilisé pour une élution par gradient dans ladite chromatographie sur colonne de gel de silice, le rapport volumique de n-hexane à l'acétate d'éthyle est compris entre 50:1 et 1:1, de préférence de 40:1 / 10:1 / 5:1 pour l'élution par gradient.

8. Composition comprenant un composé de Formule A selon l'une quelconque des revendications 1 à 5, **caractérisée en ce que**, ladite composition est une huile, une graisse ou un agent micro-sphérique.

9. Composition comprenant un composé de Formule A selon l'une quelconque des revendications 1 à 5, ou composition selon la revendication 8, destinée à être utilisée dans le traitement du cancer ; ledit médicament est de préférence utilisé pour inhiber des cellules cancéreuses avec des récepteurs d'oestrogène, en particulier de préférence utilisé pour inhiber les cellules du cancer du sein.

10. Utilisation d'une composition comprenant un composé de Formule A selon l'une quelconque des revendications 1 à 5 ou composition selon la revendication 8 destiné à la fabrication d'un médicament dans le traitement du cancer ; ledit médicament est de préférence utilisé pour inhiber des cellules cancéreuses avec des récepteurs d'oestrogène, en particulier de préférence utilisé pour inhiber les cellules du cancer du sein.
